# EUROPEAN PATENT APPLICATION

(11) **EP 1 714 657 A1**
(43) Date of publication of application: **25.10.2006**
(21) Application number: 05709637.2
(22) Date of filing: 02.02.2005
(51) Int. Cl.: A61K 33/14, A61K 31/7004, A61K 9/14, A61P 7/08, A61J 3/06

(54) **SOLID FORMULATION FOR DIALYSIS AND PROCESS FOR PRODUCING THE SAME**

(30) Priority: 09.02.2004 JP 2004032588
(71) Applicant: NIPRO CORPORATION, Osaka-shi, Osaka 531-8510 (JP)
(72) Inventor: KAI, Toshiya, Nipro Corporation, Osaka-shi, Osaka 5318510 (JP); KATAYAMA, Naohisa, c/o Nipro Corporation, Osaka-shi, Osaka 5318510 (JP); YOKOE, Jun-ichi, c/o Nipro Corporation, Osaka-shi, Osaka 5318510 (JP); SATO, Makoto, c/o Nipro Corporation, Osaka-shi, Osaka 5318510 (JP)
(74) Representative: Albrecht, Thomas
(86) International application number: PCT/JP2005/001517
(87) International publication number: WO 2005/074948

(57) **Abstract**

The present invention relates to a solid formulation for dialysis and a method for producing the same. The formulation is a mixture of: first particles having a coating layer containing magnesium chloride and sodium acetate on surfaces of sodium chloride particles; and second particles having a coating layer containing calcium chloride and sodium acetate on the surfaces of sodium chloride particles, at least one of the coating layers of the first and second particles further including potassium chloride. In the formulation of the present invention, aggregation or agglomeration between particles due to moisture absorption thereof can be suppressed. Besides, the content of each component in the formulation becomes uniform, so as to quickly dissolve in water when used. In addition, the formulation of the present invention is an extremely stable solid formulation in which decomposition of glucose hardly occurs even when a formulation containing electrolyte components and acetic acid further contains glucose.

## Description

### Technical Field

The present invention relates to a solid formulation for bicarbonate dialysis. More specifically, the present invention relates to a solid formulation for dialysis, which is obtained by mixing: first particles that contain magnesium chloride on surfaces of core particles of sodium chloride while not containing calcium chloride; and second particles that contain calcium chloride on surfaces of a core particle of sodium chloride while not containing magnesium chloride.

The solid formulation for dialysis of the present invention is used for a double-preparation type bicarbonate solid formulation for dialysis, which is formed of a formulation that contains sodium bicarbonate and a formulation that contains an electrolyte other than sodium bicarbonate, acid, and glucose, or a triple-preparation type bicarbonate solid formulation for dialysis, which is formed of a formulation that contains sodium bicarbonate, a formulation that contains an electrolyte other than sodium bicarbonate and acid, and a formulation that contains glucose.

### Background Art

When hemodialysis is carried out for a patient suffering from weakened kidney function, the blood of the patient is cleaned in an artificial kidney. Generally, a dialysate is perfused in this artificial kidney and brought into contact via a dialysis membrane with the blood of the patient so that wastes in the blood are transferred to the dialysate. As the dialysate, anacetate dialysate has been used. However, the dialysate causes unpleasant conditions (e.g.,headachesand hypotension)during dialysis. In recent years, therefore, a dialysate using sodium bicarbonate that relieves these unpleasant conditions (i.e., a sodium bicarbonate dialysate) has come into use.

The bicarbonate dialysate generates insoluble carbonate when sodium hydrogencarbonate reacts with calcium chloride or magnesium chloride, so it cannot be stored as a single solution. The bicarbonate dialysate is formulated by preparing different solutions: a solution containing potassium, calcium, and magnesium ions and a solution of sodium hydrogencarbonate.

The bicarbonate dialysate is usually prepared from two different dialysate formulations, that is, a liquid formulation containing electrolytes(e.g.,sodium chloride, potassium chloride, calcium chloride, magnesium chloride, and sodium acetate) and a pH-adjusting agent (e.g., acetic acid) (hereinafter, referred to as "liquid formulation A"), and a liquid formulation containing sodium hydrogencarbonate (sodium bicarbonate) (hereinafter, referred to as "liquid formulation B"). These dialysate formulations (liquid formulation A or B) may contain a sugar component such as glucose, or may contain no sugar component but be mixed with another formulation containing a sugar component instead.

Heretofore, the liquid compositions A and B have been sold in the form of a concentrated solution prepared at a predetermined concentration and used by a customer after being diluted with water. However, about 300 liters of a dialysate is necessary for carrying out a single dialysis for one patient, and thus, in conducting dialysis treatment for a large number of patients, a large quantity of the concentrated solution must be used, which requires dilution with water. Accordingly, many cases arise where a formulation obtained by pulverizing the components of formulation B (hereinafter, referred to as formulation B) is used to reduce the burden on those who prepare the dialysate and to reduce storage space. In this connection, commercially available are a double-preparation type solid formulation for bicarbonate dialysis that contains a formulation obtained by freeze-drying the liquid formulation A (hereinafter, referred to as formulation A) and formulation B, or atriple-preparation type solid formulation for bicarbonate dialysis that contains a glucose powder as an additional formulation in addition to the formulations A and B. Further, in recent years, a double-preparation type formulation has been developed in which glucose is added to the formulation A.

It is reported that a solid formulation for bicarbonate dialysis obtained by pulverizing the formulation A can be produced by a method of: adding an aqueous suspension of electrolytes such as potassium chloride, calcium chloride, and magnesium chloride to sodium chloride in a stirring granulator; mixing the whole while stirring under heat; adding sodium acetate to the resultant mixture and mixing them; heating the whole at 50°C or more to dissolve the sodium acetate; and mixing the mixture with acetic acid (JP-A-6-178802 and EP-A-602921).

In addition, there is disclosed a method for producing formulation A by a fluid bed granulation process that involves spraying an aqueous solution containing electrolyte components, such as potassium chloride, calcium chloride, magnesium chloride, and sodium acetate, onto sodium chloride particles fluidized in a fluid-bed granulator to allow the particles to be increased in weight, to thereby obtain a granulated product, and adding acetic acid to the granulated product and then mixing them together (JP-A-2-311418, EP-A-399918).

As a pulverized solid formulation for bicarbonate dialysis of a double-preparation type in which formulation A contains glucose, there is disclosed a formulation for dialysis that contains two compositions, that is, one powdery solid formulation (formulation A) containing an electrolyte other than sodium bicarbonate, glucose, and acetic acid, and the other powdery solid formulation (formulation B) containing only sodium bicarbonate, sodium bicarbonate and sodium acetate, or sodium bicarbonate and glucose. Among these formulations for dialysis, the formulation A can be produced by, for example, a dry process where an electrolyte other than sodium bicarbonate and glucose are mixed while stirring by a stirring mixer, pulverized by a pulverizer, and subsequently granulated in a dry granulator, followed by blending and mixing with acetic acid. Another example of a method for producing the formulation A is a fluid-bed granulation process where sodium chloride and glucose are mixed in advance by a stirring mixer, fluidized in a fluid-bed granulator, and subsequently granulated while being sprayed with aqueous solutions of potassium chloride, calcium chloride, magnesium chloride, and sodium acetate, followed by a step of mixing them with acetic acid (JP-A-2-311419, JP-A-3-38527, EP-A-602014).

### Problems to be solved by the Invention

The formulations for dialysis have disadvantages in that magnesium chloride and calcium chloride each having deliquescence properties exist together in a granulated product and are in contact with each other to increase moisture-absorption characteristics, so the granulated materials are apt to be agglomerated owing to an influence of moisture at the time of production or storage. When the agglomerated of granulated product occurs, unevenness of the components in the composition may occur, so the rate of dissolution becomes slower and a uniform dialysate becomes difficult to be prepared. For this reason, there is a disadvantage in that a specific packaging configuration such as a packaging configuration using aluminum laminated film or the like, for keeping the formulation completely away from moisture and preventing it from agglomerating, may be required, and that a dialysate should be prepared quickly by dissolving in water after being opened. When the formulation A contains glucose, there is a disadvantage in that the decomposition of glucose can be accelerated by agglomeration of a granulated product containing calcium chloride and magnesium chloride.

### Means for solving the Problem

The inventors of the present invention have studied about a formulation for dialysis, in which electrolyte components do not agglomerate and in which glucose is not decomposed, and found out that as a solid formulation for dialysis containing electrolytes other than sodium bicarbonate (formulation A), a solid formulation for dialysis (formulation A) is produced by independently granulating first particles having magnesium chloride on the surfaces of core particles of sodium chloride while not having calcium chloride, and second particles having calcium chloride on the surfaces of core particles of sodium chloride while not having magnesium chloride, and then mixing the particles with acid, thereby causing no agglomeration of electrolyte components and no decomposition of glucose even after the addition of glucose. According to those findings, the inventors of the present invention have studied in various ways to complete the present invention.

That is, the present invention relates to:
(1) a solid formulation for dialysis, which contains sodium chloride, potassium chloride, calcium chloride, magnesium chloride, and sodium acetate as electrolyte components, including a mixture of:
   first particles having a coating layer containing electrolyte components except calcium chloride, on surfaces of sodium chloride particles; and
   second particles having a coating layer containing electrolyte components except magnesium chlorine, on the surfaces of sodium chloride particles,
(2) a solid formulation for dialysis according to item (1), including a mixture of:
   the first particles having a coating layer containing magnesium chloride and sodium acetate on the surfaces of sodium chloride particles; and
   the second particles having a coating layer containing calcium chloride and sodium acetate on the surfaces of sodium chloride particles,
   in which at least one of the coating layers of the first particles and the second particles further contains potassium chloride,
(3) a solid formulation for dialysis according to item (1) or (2), including a mixture of:
   the first particles having a coating layer containing potassium chloride, magnesium chloride, and sodium acetate on the surfaces of sodium chloride particles; and
   the second particles having a coating layer containing calcium chloride and sodium acetate on the surfaces of sodium chloride particles,
   in which the coating layer of the second particles may further contain potassium chloride,
(4) a solid formulation for dialysis according to any one of items (1) to (3), further including third particles containing glucose,
(5) a solid formulation for dialysis according to any one of items (1) to (3), further including acetic acid,
(6) a solid formulation for dialysis according to any one of items (1) to (3), including:
   the first particles having a coating layer containing potassium chloride, magnesium chloride, and sodium acetate on the surfaces of sodium chloride particles;
   the second particles having a coating layer containing potassium chloride, calcium chloride, and sodium acetate on the surface of sodium chloride particles;
   the third particles containing glucose; and
   acetic acid,
(7) a method for producing a solid formulation for dialysis containing sodium chloride, potassium chloride, calcium chloride, magnesium chloride, and sodium acetate as electrolyte components, characterized by uniformly mixing:
   first particles having a coating layer containing electrolyte components except calcium chloride, on surfaces of sodium chloride particles;
   second particles having a coating layer containing electrolyte components except magnesium chloride, on the surfaces of sodium chloride particles;
   acetic acid; and optionally
   third particles containing glucose,
(8) a method for producing a solid formulation for dialysis, characterized by uniformly mixing:
   first particles having a coating layer containing magnesium chloride and sodium acetate on surfaces of sodium chloride particles;
   second particles having a coating layer containing calcium chloride and sodium acetate on the surfaces of sodium chloride particles, in which at least one of the coating layers of the first particles and the second particles further contains potassium chloride;
   acetic acid; and optionally
   third particles containing glucose,
(9) a method for producing a solid formulation for dialysis according to item (7) or (8), in which acetic acid is added to each of the first particles and the second particles before the whole is uniformly mixed, and
(10) a method for producing a solid formulation for dialysis according to item (7) or (8), in which the first particles and the second particles are added to the third particles and the acetic acid is then added to uniformly mix the whole.

### Effects of the Invention

In the solid formulation for dialysis of the present invention (hereinafter, also abbreviated as "the present formulation"), calcium chloride and magnesium chloride which are highly hygroscopic are present as different particles (e.g., granules), so the aggregation or agglomeration between particles due to moisture absorption thereof can be prevented, compared with the conventional formulation in which calcium chloride and magnesium chloride coexist in the same particles. Besides, the content of each component in the formulation of the present invention becomes uniform, so it can be quickly dissolved in water when used. Further, the formulation of the present invention containing glucose is extremely stable because the decomposition of glucose and coloring are prevented. The formulation of the present invention is stable and can be stored for a long period of time and is provided as a dry powder, so it can be handled very easily.

### Best Mode for carrying out the Invention

The first particles of the formulation of the present invention have a coating layer on the surfaces of sodium chloride particles. The coating layer contains an electrolyte component other than calcium chloride and also contains potassium chloride, magnesium chloride, and sodium acetate. When potassium chloride is retained in the coating layer of the second particles described below, potassium chloride does not need to be added to the coating layer of the first particles, although it is preferable. The coating layer of the first particles may further contain sodium chloride as an electrolyte component.

The average particle size of the first particles is about 75 to 1,700 µm, preferably about 100 to 1,000 µm. A thickness of the coating layer is not specifically limited as long as the thickness is enough to guarantee the uniformity of the electrolyte components. Also, there is no need to completely coat the surface of the core particles.

For the core particles of sodium chloride forming the first particle, a powder, particularly a powdery crystal, is used. Typically, the average particle size thereof is about 60 to 1,500 µm, preferably about 75 to 500 µm. The core particle of sodium chloride may further contain one or two or more kinds of electrolytes, such as magnesium chloride, potassium chloride, and sodium acetate, in addition to sodium chloride by means of adhesion, mixture, or the like.

Magnesium chloride hexahydrate or the like may be used as the above-mentioned magnesium chloride. Examples of the above-mentioned sodium acetate to be used include sodium acetic anhydride and sodium acetate trihydrate.

The second particles of the formulation of the present invention have a coating layer on the surfaces of sodium chloride particles. The coating layer contains an electrolyte component other than magnesium chloride and also contains potassium chloride, calcium chloride, and sodium acetate. When potassium chloride is retained in the coating layer of the first particles, potassium chloride does not need to be added to the coating layer of the second particles, although it ispreferable. The coating layer of the second particles may further contain sodium chloride as an electrolyte

The average particle size of the second particles is about 75 to 1,700 µm, preferably about 100 to 1, 000 µm. A thickness of the coating layer is not specifically limited as long as the thickness is enough to guarantee the uniformity of the electrolyte components. Also, there is no need to completely coat the surface of a core particle. The average particle size of the first particles may be the same as or different from that of the second particles. However, it is preferable that average particle sizes thereof are almost equal to each other.

For the core particles of sodium chloride of the second particles, a powder, particularly a powdery crystal, is used as in the case of the core particles of the first particles. Typically, the average particle size thereof is about 60 to 1, 500 µm, preferably about 75 to 500 µm. The core particles of sodium chloride may further contain one or two or more kinds of calcium chloride, potassium chloride, and sodium acetate in addition to sodium chloride by means of adhesion, mixture, or the like.

The coating layer may further contain sodium chloride in addition to the above-mentioned electrolytes. Examples of the above-mentioned calcium chloride to be used include calcium chloride dihydrate, calcium chloride monohydrate, and calcium chloride anhydride. Examples of the above-mentioned sodium acetate to be used include sodium acetic anhydride and sodium acetate trihydrate.

The third particles of the formulation of the present invention may employ glucose-containing particles such as a glucose powder, a granulated product having a coating layer of glucose on glucose core particles, a granulated product provided as a mixture of a glucose powder and a sodium chloride powder, and the like. The third particles are preferably a glucose powder. The average particle size of the glucose-containing particles is preferably adjusted to substantially the same size as those of the first and second particles. When the powdery crystal of glucose is used as it is without being granulated, the average particle size thereof is preferably about 100 to 1,000 µm.

The formulation of the present invention is a solid formulation for dialysis, which is provided as a mixture of first particles having a coating layer containing magnesium chloride and sodium chloride on the surfaces of sodium chloride particles, and second particles having a coating layer containing calcium chloride and sodium acetate on the surfaces of sodium chloride particles, in which at least one of the coating layers of the first and second particles further contains potassium chloride. The formulation of the present invention is preferably a solid formulation for dialysis, which is provided as a mixture of first particles having a coating layer containing potassium chloride, magnesium chloride, and sodium acetate, and second particles having a coating layer containing calcium chloride and sodium acetate, in which the coating layer of the second particles may further contain potassium chloride. The formulation of the present invention is more preferably a solid formulation for dialysis, which is provided as a mixture of first particles having a coating layer containing potassium chloride, magnesium chloride, and sodium acetate on the surfaces of sodium chloride particles, and second particles having a coating layer containing potassium chloride, calcium chloride, and sodium acetate on the surfaces of sodium chloride particles.

Specific examples of the formulation of the present invention include:
(1) first particles having a coating layer containing magnesium chloride, sodium acetate, and potassium chloride, second particles having a coating layer containing calcium chloride, sodium acetate, and potassium chloride, and acetic acid;
(2) first particles having a coating layer containing magnesium chloride, sodium acetate, and potassium chloride, second particles having a coating layer containing calcium chloride and sodium acetate, and acetic acid;
(3) first particles having a coating layer containing magnesium chloride and sodium acetate, second particles having a coating layer containing calcium chloride, sodium acetate, and potassium chloride, and acetic acid;
(4) first particles having a coating layer containing magnesium chloride, sodium acetate, and potassium chloride, second particles having a coating layer containing calcium chrolide, sodium acetate, and potassium chloride, a glucose powder, and acetic acid;
(5) first particles having a coating layer containing magnesium chloride, sodium acetate, and potassium chloride, second particles having a coating layer containing calcium chloride and sodium acetate, a glucose powder, and acetic acid; and
(6) first particles having a coating layer containing magnesium chloride and sodium acetate, second particles having a coating layer containing calcium chloride, sodium acetate, and potassium chloride, a glucose powder, and acetic acid.

The solid formulation for dialysis of the present invention containing sodium chloride, potassium chloride, calcium chloride, magnesium chloride, and sodium acetate as electrolyte components can be produced by uniformly mixing first particles having a coating layer containing electrolyte components except calcium chloride on the surfaces of sodium chloride particles, second particles having a coating layer containing electrolyte components except magnesium chloride on the surfaces of sodium chloride particles, acetic acid, and optionally third particles containing glucose.

Preferably, the formulation of the present invention can be produced by uniformly mixing first particles having a coating layer containing calcium chloride, magnesium chloride, and sodium acetate on the surfaces of sodium chloride particles, second particles having a coating layer containing calcium chloride, sodium acetate, and optionally potassium chloride on the surfaces of sodium chloride particles, acetic acid, and optionally third particles containing glucose. The solid formulation for dialysis of the present invention can also be produced in the same manner as described above by allowing the coating layer of the second particles to contain potassium chloride while the coating layer of the first particles do not contain potassium chloride.

The first and second particles of the formulation of the present invention can be produced by any of methods known in the art, for example, any of general granulation processes such as a fluidized bed spray granulation process, a fluidized bed granulation process, a rolling and agitating fluidized bed granulation process and an agitation granulation process.

For instance, in a device for rolling-agitation fluid-bed granulation, first particles can be produced by spraying an aqueous solution of a mixture containing electrolytes such as sodium chloride, potassium chloride, magnesium chloride, and sodium acetate onto rolling and fluidizing core particles of sodium chloride, thereby forming the coating layer of the electrolyte on the surface of the particles of sodium chloride.

Further, in a device for rolling-agitation fluid-bed granulation, second particles can be produced by spraying an aqueous solution of a mixture containing electrolytes such as sodium chloride, potassium chloride, calcium chloride, and sodium acetate onto rolling and fluidizing particles of sodium chloride , thereby forming the coating layer of the electrolyte on the surface of the particles of sodium chloride.

In a rolling-agitation fluid-bed granulation process of the first and second particles, the rate of airflow is preferably about 1 to 150 m³/min. An intake-air temperature is about 40 to 100°C, and an exhaust-air temperature from a rolling-agitation fluid-bed granulator is about 25 to 70°C. The time required for granulation is generally about 30 minutes to 1 hour, but is not limited thereto because of variations of the time depending on the conditions of production scale, the intake-air temperature, the exhaust temperature, and the like.

An aqueous solution of electrolytes used for the formation of a coating layer on the core particles of sodium chloride is generally used at a solids concentration of about 15 to 50% by weight.

The third particles can be produced by any of granulation processes such as a fluid-bed granulation process, a rolling-agitation fluid-bed granulation process, a dry granulation process, and an agitation-granulation process, or by adjusting the conditions or the like of screening and crystallization during the production of glucose. When glucose-containing particles are granulated, for example, an aqueous glucose solution is sprayed on a core particle of glucose, followed by granulation with a general granulation process such as a fluid-bed granulation process, a rolling-agitation fluid-bed granulation process, or the like.

The first to third particles are preferably produced by a rolling-agitation fluid-bed granulation process.

The first to third particles are granulated into granules or powders each having average particle sizes of about 75 µm to 1,700 µm, preferably about 100 µm to 1,000 µm. Therefore, each of the compositions can be uniformly mixed to obtain a solid formulation for dialysis with high uniformity. When the glucose provided as the above-mentioned third particles is used together with other components but not as a granulated product, the average particle size of glucose may be preferably adjusted in the range of about 100 to 1,000 µm.

The formulation of the present invention can be produced by adding the first particles, the second particles, the third particles such as a glucose granulated product if needed, and also acetic acid as a pH regulator, to uniformly mix them together.

Generally, the mixture of these particles and the mixture of acetic acid with these particles, can be carried out by using a mixer such as a V-type mixer, a container type mixer, andanagitation mixer.

The order of adding the first, second, and third particles and acetic acid is not specifically limited, but preferably the first and second particles are added to a mixer, and then acetic acid is added and mixed. Alternatively, when the third particles are added, the third particles are added to a mixer first and then the first and second particles are added thereto, and acetic acid is added to the first and second particles, to thereby sufficiently mix in a uniform manner. The order of adding the first to third particles and acetic acid at a time of mixing prevents the decomposition of glucose caused by direct contact between acetic acid and glucose in the mixture. The time period required for mixing depends on an amount to be mixed, but generally is about 10 to 30 minutes. The production method for the present invention allows the formulation of the present invention in the form of a dry and stable powder to be obtained.

The formulation of the present invention is mixed with another solid formulation (formulation B) containing sodium bicarbonate at a predetermined mixing ratio when required, and then dissolved in water, thereby preparing a dialysate. Alternatively, the formulation of the present invention and another solid formulation containing sodium bicarbonate each may be independently dissolved in water to prepare two different aqueous solutions, followed by mixing the solutions to prepare a dialysate. In addition, one of the formulation of the present invention and sodium bicarbonate may be dissolved in water first to prepare an aqueous solution and then the other formulation thereof may be dissolved in the aqueous solution, thereby preparing a dialysate.

When the formulation of the present invention and sodium bicarbonate are dissolved together in water to prepare a dialysate, the ionic concentration of electrolyte components of the bicarbonate dialysate are as follows. In addition, the pH level of the bicarbonate dialysate is preferably about 7.2 to 7.4.

| | |
|---|---|
| Na⁺ | 120 to 150 mEq/L |
| K⁺ | 0.5 to 3 mEq/L |
| Ca²⁺ | 1.5 to 4.5 mEq/L |
| Mg²⁺ | 0.1 to 2.0 mEq/L |
| Cl⁻ | 90 to 135 mEq/L |
| CH₃COO⁻ | 5 to 15 mEq/L |
| HCO₃⁻ | 20 to 35 mEq/L. |
| When glucose is included | 0.5 to 2.5 g/L. |

The formulation of the present invention is a composition with uniform quality. Thus, when a certain amount is taken from any part of the formulation and is dissolved in a certain amount of water, the ionic concentrations of the respective electrolyte components are always constant.

Hereinafter, the present invention will be described in more detail with reference to examples, comparative examples, and test examples.

In the examples and comparative examples, the term "magnesium chloride" means "magnesium chloride hexahydrate" and the term "calcium chloride" means "calcium chloride dihydrate".

### Example 1

An aqueous solution was prepared by uniformly dissolving 32. 0 parts by weight of magnesium chloride and 103.35 parts by weight of anhydrous sodium acetate in 323.2 parts by weight of purified water. The above-mentioned aqueous solution was sprayed onto 984.9 parts by weight of sodium chloride with an average particle size of 300 µm which was being fluidized in a rolling fluid-bed granulator (MP-01, manufactured by Powrex), under the conditions of an intake-air temperature of 80°C and a rotor speed of 300 rpm, and then dried, thereby producing a granulated product in a granular form as first particles having an average particle size of 500 µm.

Next, an aqueous solution was prepared by uniformly dissolving 47.0 parts by weight of potassium chloride, 69.5 parts by weight of calcium chloride, and 103.35 parts by weight of sodium acetate anhydride in 525.4 parts by weight of purified water. The above-mentioned aqueous solution was sprayed onto 984.9 parts by weight of sodium chloride with an average particle size of 300 µm, which was being fluidized in a rolling fluid-bed granulator (MP-01, manufactured by Powrex), under the conditions of an intake-air temperature of 80°C, a rotor speed of 300 rpm, and an exhaust temperature of 40 to 50°C, and then dried, thereby producing a granulated product in a granular form as second particle having an average particle size of 500 µm.

In a mixture of the first and second particles, 42.0 parts by weight of acetic acid was added and then the whole was mixed sufficiently by means of a V-type mixer, thereby producing the pharmaceutical agent of the present invention.

### Example 2

An aqueous solution was prepared by uniformly dissolving 47.0 parts by weight of potassium chloride, 32.0 parts by weight of magnesium chloride, and 103.35 parts by weight of sodium acetate anhydride in 435.8 parts by weight of purified water. The above-mentioned aqueous solution was sprayed onto 984.9 parts by weight of sodium chloride with an average particle size of 300 µm, which was being fluidized in a rolling fluid-bed granulator (MP-01, manufactured by Powrex), under the conditions of an intake-air temperature of 80°C, a rotor speed of 300 rpm, an exhaust temperature of 40 to 50°C, and then dried, thereby producing a granulated product in a granular form as first particles having an average particle size µm. Next, an aqueous solution was prepared by uniformly dissolving 69. 5 parts by weight of calcium chloride and 103. 35 parts by weight of sodium acetate anhydride in 413 parts by weight of purified water. The above-mentioned aqueous solution was sprayed onto 984.9 parts by weight of sodium chloride with an average particle size of 300 µm, which was being fluidized in a rolling fluid-bed granulator (MP-01, manufactured by Powrex), under the conditions of an intake-air temperature of 80°C and a rotor speed of 300 rpm, and then dried, thereby producing a granulated product in a granular form as second particles having an average particle size of 500 µm. In a mixture of the first and second particles, 42.0 parts by weight of acetic acid was added and then the whole was mixed sufficiently by means of a V-type mixer, thereby producing the p

### Example 3

First and second particles were obtained in the same way as that of Example 1 (each of the average particle sizes was about 500 µm).

Separately, 1, 000 parts by weight of an aqueous glucose solution (25 w/w%) was prepared. Then, 1,000 parts by weight of glucose powder having an average particle size of 180 µm was placed in a rolling fluid-bed granulator (MP-01, manufactured by Powrex). Then, onto the fluidized glucose particles, 500 parts by weight of the above-mentioned aqueous glucose solution was sprayed under the conditions of an intake-air temperature of 60°C, a rotor speed of 300 rpm, and an exhaust temperature of 37 to 42°C, thereby obtaining a glucose granulated product of 450 µm in average particle size (third granulated product). In a mixture of 315 parts by weight of the glucose particles and the first and second particles, 42.0 parts by weight of acetic acid was added, and then the whole was mixed by means of a V-type mixer, thereby producing the formulation of the present invention.

### Example 4

A glucose powder was sieved to prepare a powder in the range of 500 to 850 µm. The formulation of the present invention was produced by carrying out the same mixing procedure as that of Example 3 except that 315 parts by weight of glucose was used.

### Comparative Example 1

An aqueous solution was prepared by completely dissolving 47.0 parts by weight of potassium chloride, 69.5 g parts by weight of calcium chloride, 32.0 parts by weight of magnesium chloride, and 206.7 parts by weight of sodium acetate anhydride in 848.8 parts by weight of purified water. The above-mentioned aqueous solution was sprayed onto 1,969.8 parts by weight of sodium chloride with an average particle size of 300 µm, which was being fluidized in a rolling fluid-bed granulator (MP-01, manufactured by Powrex), under the conditions of an intake-air temperature of 80°C, a rotor speed of 300 rpm, and an exhaust temperature of 40 to 50°C, and then dried, thereby producing a granulated product in a granular form as first particles having an average particle size of 600 µm. The obtained granulated product was added with 42.0 parts by weight of acetic acid and then the whole was mixed by means of a V-type mixer, thereby producing a formulation.

### Comparative Example 2

A granulated product in a granular form obtained in the same way as that of Comparative Example 1, 315 parts by weight of glucose having particle sizes of 500 µm to 850 µm obtained by the same sieving process as the one described in Example 4, and 42.0 parts by weight of acetic acid were mixed by means of a V-type mixer, thereby producing a formulation.

### Test Example 1: Determination of the contents of components

From the formulations obtained from the above-mentioned Examples 1 to 4, samples under test were collected three times at an amount of 10 grams from different portions. Each of the samples was then dissolved in water to prepare a 50-mL aqueous solution, followed by determination of the content of each component in the aqueous solution. The percentage (%) and the variation coefficient (CV) (%) of the average value of the content of the component determined with respect to theoretically obtained values are shown in Table 1.

It should be noted that the contents of sodium and potassium were determined by means of a flame photometer, the contents of calcium and magnesium were determined by means of ion exchange chromatography, the content of acetic acid was determined by means of HPLC-UV, the content of chlorine was determined by a silver-nitrate titrimetric process, and the content of glucose was determined by means of a polarimeter.

Table 1 Contents of components

| | Na | K | Ca | Mg | Acetic acid | Cl | Glucose |
|---|---|---|---|---|---|---|---|
| Example 1 | 101.2 ± 0.32 | 100.4 ± 0.29 | 99.6 ± 0.47 | 99.8 ± 0.21 | 99.3 ± 0.39 | 101.5 ± 0.32 | - |
| Example 2 | 100.7 ± 0.32 | 99.6 ± 0.43 | 99.4 ± 0.35 | 99.4 ± 0.22 | 99.2 ± 0.27 | 101.3 ± 0.41 | - |
| Example 3 | 100.6 ± 0.34 | 100.6 ± 0.41 | 99.9 ± 0.39 | 99.6 ± 0.12 | 98.9 ± 0.45 | 101.7 ± 0.21 | 99.7 ± 0.62 |
| Example 4 | 101.1 ± 0.37 | 100.6 ± 0.25 | 100.2 ± 0.21 | 100.2 ± 0.22 | 98.5 ± 0.32 | 101.2 ± 0.40 | 99.5 ± 0.68 |

| | | | | | | | |
|---|---|---|---|---|---|---|---|
| (average value ± CV%) | | | | | | | |

As shown in Table 1, all of samples under test showed satisfactory content of each component and also high uniformity.

### Test Example 2: Stability test

Samples under test of 50 g each in a filling amount, obtained from formulations of Example 1, Example 2, and Comparative Examples 1 and 2, were packed in aluminum packages and polyethylene terephthalate/silica-deposited polyethylene terephthalate/low-density polyethylene laminate (PET-GL-LDPE) packing packages, and then a stability test was carried out at 40°C, 75%RH. After 1 month, 10-g parts were collected three times from different portions of each sample. Then, each sample was dissolved in water to prepare a 50-mL aqueous solution, followed by determination of the content of each component in the aqueous solution. Separately, at starting, formulations in aluminum packages and PET-GL-LDPE packages each containing 50 mg of each sample were prepared, and 10-g of samples were collected three times from different portions of each sample right after the filling, followed by determination of each content of the sample in a similar manner. The percentage (%) and the variation coefficient (CV)(%) of the average value of the content of the component determined with respect to theoretically obtained values are shown in Tables 2 and 3 together with the characteristics of each sample.

It should be noted that the contents of sodium and potassiumwere determined by means of a flame photometer, the contents of calcium and magnesium were determined by means of ion exchange chromatography, the content of acetic acid was determined by means of HPLC-UV, the content of chlorine was determined by a silver-nitrate titrimetric process, and the content of glucose was determined by means of a polarimeter.

Table 2: Stability of powdery dialysis agent (aluminum package)

| | | | Start | After 1 month |
|---|---|---|---|---|
| Example 1 | Aspects | | White powder | White powder |
| | Contents | Na | 101.2 ± 0.32 | 101.0 ± 0.29 |
| | | K | 100.4 ± 0.29 | 100.1 ± 0.34 |
| | | Ca | 99.6 ± 0.47 | 99.6 ± 0.51 |
| | | Mg | 99.8 ± 0.21 | 100.1 ± 0.31 |
| | | Acetic acid | 99.3 ± 0.39 | 99.1 ± 0.41 |
| | | Cl | 101.5 ± 0.32 | 101.3 ± 0.32 |
| | | Glu | - | - |
| Example 3 | Aspects | | White powder | White powder |
| | Contents | Na | 100.6 ± 0.34 | 101.2 ± 0.37 |
| | | K | 100.6 ± 0.41 | 100.2 ± 0.37 |
| | | Ca | 99.9 ± 0.39 | 99.6 ± 0.43 |
| | | Mg | 99.6 ± 0.12 | 99.4 ± 0.32 |
| | | Acetic acid | 98.9 ± 0.45 | 98.7 ± 0.39 |
| | | Cl | 101.7 ± 0.21 | 101.4 ± 0.32 |
| | | Glu | 99.7 ± 0.62 | 99.4 ± 0.51 |
| Comparative example 1 | Aspects | | White powder | White powder |
| | Contents | Na | 101.3 ± 0.26 | 101.5 ± 0.37 |
| | | K | 100.7 ± 0.41 | 100.1 ± 0.35 |
| | | Ca | 99.4 ± 0.36 | 99.3 ± 0.46 |
| | | Mg | 99.8 ± 0.58 | 99.5 ± 0.47 |
| | | Acetic acid | 99.1 ± 0.52 | 99.5 ± 0.36 |
| | | Cl | 101.4 ± 0.46 | 100.9 ± 0.50 |
| | | Glu | - | - |
| Comparative example 2 | Aspects | | White powder | White powder |
| | Contents | Na | 100.6 ± 0.46 | 100.6 ± 0.41 |
| | | K | 100.4 ± 0.36 | 100.5 ± 0.39 |
| | | Ca | 100.1 ± 0.44 | 99.9 ± 0.46 |
| | | Mg | 99.8 ± 0.50 | 99.7 ± 0.44 |
| | | Acetic acid | 98.0 ± 0.59 | 98.4 ± 0.47 |
| | | Cl | 101.6 ± 0.56 | 101.3 ± 0.53 |
| | | Glu | 99.4 ± 0.69 | 99.5 ± 0.71 |

| | | | | |
|---|---|---|---|---|
| "Glu" means glucose (hereinafter, the same holds true) (average value ± CV%) | | | | |

Table 3: Stability of powdery dialysis agent (PET-GL-LDPE packing package)

| | | | Start | After 1 month |
|---|---|---|---|---|
| Example 1 | Aspects | | White powder | White powder |
| | Contents | Na | 101.2 ± 0.32 | 101.3 ± 0.42 |
| | | K | 100.4 ± 0.29 | 100.1 ± 0.36 |
| | | Ca | 99.6 ± 0.47 | 100.4 ± 0.37 |
| | | Mg | 99.8 ± 0.21 | 99.6 ± 0.31 |
| | | Acetic acid | 99.3 ± 0.39 | 99.6 ± 0.32 |
| | | Cl | 101.5 ± 0.32 | 101.1 ± 0.30 |
| | | Glu | - | - |
| Example 3 | Aspects | | White powder | White powder |
| | Contents | Na | 100.6 ± 0.34 | 101.0 ± 0.29 |
| | | K | 100.6 ± 0.41 | 100.2 ± 0.34 |
| | | Ca | 99.9 ± 0.39 | 100.2 ± 0.34 |
| | | Mg | 99.6 ± 0.12 | 99.6 ± 0.29 |
| | | Acetic acid | 98.9 ± 0.45 | 99.1 ± 0.33 |
| | | Cl | 101.7 ± 0.21 | 101.4 ± 0.19 |
| | | Glu | 99.7 ± 0.62 | 99.5 ± 0.67 |
| Comparative example 1 | Aspects | | White powder | White, slightly agglomerated |
| | Contents | Na | 101.3 ± 0.26 | 101.4 ± 0.64 |
| | | K | 100.7 ± 0.41 | 99.4 ± 1.21 |
| | | Ca | 99.4 ± 0.36 | 100.2 ± 3.04 |
| | | Mg | 99.8 ± 0.58 | 99.4 ± 3.32 |
| | | Acetic acid | 99.1 ± 0.52 | 99.3 ± 1.12 |
| | | Cl | 101.4 ± 0.46 | 101.7 ± 0.72 |
| | | Glu | - | - |
| Comparative example 2 | Aspects | | White powder | Yellow, agglomerated |
| | Contents | Na | 100.6 ± 0.46 | 101.4 ± 0.82 |
| | | K | 100.4 ± 0.36 | 99.3 ± 2.36 |
| | | Ca | 100.1 ± 0.44 | 98.7 ± 2.31 |
| | | Mg | 99.8 ± 0.50 | 101.2 ± 4.50 |
| | | Acetic acid | 98.0 ± 0.59 | 98.4 ± 1.59 |
| | | Cl | 101.6 ± 0.56 | 101.2 ± 0.96 |
| | | Glu | 99.4 ± 0.69 | 98.4 ± 3.69 |

| | | | | |
|---|---|---|---|---|
| (average value ± CV%) | | | | |

As shown in Table 2, when the formulation was kept in a water-impermeable aluminum package, no agglomeration occurred in each sample and the content and uniformity of each component were favorable. However, as shown in Table 3, when the formulation was kept in a PET-GL-LDPE package through which moisture permeates, the samples prepared in Comparative Examples 1 and 2 agglomerated during storage owing to the influence of moisture. In addition, coloring of glucose was recognized (yellowing) in the sample of Comparative Example 2.

Owing to the influence, as is evident from the variation coefficient of the content of each component of the formulation stored for 1 month shown in Table 3, variations occurred in contents of calcium and magnesium in the samples of Comparative Examples 1 and 2, while the uniformity of glucose was decreased in the sample of Comparative Example 2.

Test Example 3: Determination of dissolution time 131.5 g of each sample under test after one month from the stability test as represented in Table 3 were weighed out and then added to 500 ml of purified water, followed by stirring with a magnetic stirrer. Then, visual observation was carried out, and the dissolution time from the time of addition was then determined.

Table 4 Dissolution time

| Sample under test | Dissolution time (sec.) |
|---|---|
| Example 1 | 400 |
| Example 3 | 380 |
| Comparative example 1 | 650 |
| Comparative example 2 | 580 |

As shown in Table 4, formulations prepared in Examples 1 and 3 showed dissolution rates of 400 seconds or less, respectively. In contrast, formulations prepared in Comparative Examples 1 and 2 had a clearly extended dissolution time and thus showed that dissolution hardly occurred because of agglomeration of the granulated product or the like.

### INDUSTRIAL APPLICABILITY

The formulation of the present invention contains highly hygroscopic calcium chloride and magnesium chloride, which are provided as particles separated from others to thereby prevent aggregation and agglomeration between the component particles. The compositional particles become uniform, so there is obtained a formulation which has excellent uniformity in each content of component in the formulation, and which easily dissolves in water when used. In the formulation of the present invention containing glucose, the decomposition and coloring of glucose is prevented, providing extremely high stability. The formulation of the present invention is a solid formulation to be used in a dialysate for bicarbonate, which can be retained for a long time and handled with ease.

## Claims

1. A solid formulation for dialysis, which contains sodium chloride, potassium chloride, calcium chloride, magnesium chloride, and sodium acetate as electrolyte components, comprising a mixture of:
first particles having a coating layer containing electrolyte components except calcium chloride, on surfaces of sodium chloride particles; and
second particles having a coating layer containing electrolyte components except magnesium chlorine, on the surfaces of sodium chloride particles.

2. A solid formulation for dialysis according to claim 1, comprising a mixture of:
the first particle having a coating layer containing magnesium chloride and sodium acetate on the surfaces of sodium chloride particles; and
the second particle having a coating layer containing calcium chloride and sodium acetate on the surfaces of sodium chloride particles,
wherein at least one of the coating layers of the first particle and the second particle further contains potassium chloride.

3. A solid formulation for dialysis according to claim 1 or 2, comprising a mixture of:
the first particle having a coating layer containing potassium chloride, magnesium chloride, and sodium acetate on the surfaces of sodium chloride particles; and
the second particle having a coating layer containing calcium chloride and sodium acetate on the surfaces of sodium chloride particles,
wherein the coating layer of the second particle may further contain potassium chloride.

4. A solid formulation for dialysis according to any one of claims 1 to 3, further comprising thrid particles containing glucose.

5. A solid formulation for dialysis according to any one of claims 1 to 3, further comprising acetic acid.

6. A solid formulation for dialysis according to any one of claims 1 to 3, comprising:
the first particle having a coating layer containing potassium chloride, magnesium chloride, and sodium acetate on surfaces of sodium chloride particles;
the second particle having a coating layer containing potassium chloride, calcium chloride, and sodium acetate on the surface of sodium chloride particle;
the third particle containing glucose; and
acetic acid.

7. A method for producing a solid formulation for dialysis containing sodium chloride, potassium chloride, calcium chloride, magnesium chloride, and sodium acetate as electrolyte components, **characterized by** uniformly mixing:
first particles having a coating layer containing electrolyte components except calcium chloride, on surfaces of sodium chloride particles;
second particles having a coating layer containing electrolyte components except magnesium chlorine, on the surfaces of sodium chloride particles;
acetic acid; and optionally
third particles containing glucose.

8. A method for producing a solid formulation for dialysis, **characterized by** uniformly mixing:
first particles having a coating layer containing magnesium chloride and sodium acetate on surfaces of sodium chloride particles;
second particles having a coating layer containing calcium chloride and sodium acetate on the surfaces of sodium chloride particles, wherein at least one of the coating layers of the first particle and the second particle contains potassium chloride;
acetic acid; and optionally
third particles containing glucose.

9. A method for producing a solid formulation for dialysis according to claim 7 or 8, wherein acetic acid is added to each of the first particle and the second particle before the whole is uniformly mixed.

10. A method for producing a solid formulation for dialysis according to claim 7 or 8, wherein the first particle and the second particle are added to the third particle and the acetic acid is then added to uniformly mix the whole.
